# EUROPÄISCHE PATENTSCHRIFT

(11) **EP 0 983 341 B1**
(45) Veröffentlichungstag und Bekanntmachung des Hinweises auf die Patenterteilung: **16.04.2003**
(21) Anmeldenummer: 98925572.4
(22) Anmeldetag: 11.05.1998
(51) Int. Cl.: C12M 3/06

(54) **VORRICHTUNG ZUM ZÜCHTEN UND/ODER BEHANDELN VON ZELLEN**
DEVICE FOR GROWING OR TREATING CELLS
DISPOSITIF POUR LA CULTURE ET/OU LE TRAITEMENT DE CELLULES

(30) Priorität: 10.05.1997 DE 19719751
(43) Veröffentlichungstag der Anmeldung: 08.03.2000
(73) Patentinhaber: Bader, Augustinus, 31275 Lehrte (DE)
(72) Erfinder: Bader, Augustinus, 31275 Lehrte (DE)
(74) Vertreter: Lorenz, Werner, Dr.-Ing.
(86) Internationale Anmeldenummer: EP9802745
(87) Internationale Veröffentlichungsnummer: WO98051779

(56) Entgegenhaltungen:
- EP-A- 0 725 134
- WO-A-90/05179
- WO-A-96/30497
- US-A- 4 661 455

## Beschreibung

Die Erfindung betrifft eine Vorrichtung zum Züchten und/oder Behandeln von Zellen.

Aus der DE 42 06 585 C2 ist eine Vorrichtung zur Massenkultur von Zellen, insbesondere von Hepatozyten auf plattenartigen Zellkulturträgern beschrieben, wobei auf einem gasdurchlässigen Zellkulturträger Zellen in einer Kollagenschicht angeordnet sind. Die Vorrichtung ist dabei sandwichartig aufgebaut, wobei mehrere Zellkulturträger mit dazwischen liegenden Kollagenschichten mit Zellen vorgesehen sind. Nachteilig bei dieser Vorrichtung ist, daß stets genau definierte Zellenkammern mit bestimmten Volumina vorhanden sind. Darüber hinaus ist es schwierig die Zellen in den Zellenkammern zu beobachten, um eventuelle Veränderungen bzw. Entwicklungszustände festzustellen.

In der DE 42 22 345 A1 ist ein Verfahren zum Züchten einer Zellart im Kokulturverfahren mit Leberzellen beschrieben, wobei eine Kultivierung von Leberzellen auf einem Träger im Sandwichverfahren erfolgt. Zwischen den Leberzellen und dem Träger ist eine erste Matrixschicht zur Verankerung der Leberzellen angeordnet und über den Leberzellen befindet sich eine zweite Matrixschicht.

In USP 5,449.617 wird ein Kulturgefäß beschrieben in dem die Kultur in starren Gehäusen durchgeführt wird. Ein Nachteil ist hierbei der hohe Anteil an Totvolumen. Um eine verbesserte und gleichmäßigere Versorgung der Zellen mit Sauerstoff zu erreichen, muß das Gerät vorzugsweise gedreht werden. Dies ist insbesonders für adhärent wachsende Zellen ein kontinuierlicher Streßfaktor. Die Erstellung von Hepatozytenkulturen nach der sog. Sandwichtechnik ist hierbei nicht längerfristig möglich, da derartige Rotationen zu einer Zerstörung der Kollagenschichten führen. Der Skale up bei derartigen Verfahren ist nur mit hohem Totvolumen möglich. Der Skale up unterliegt dadurch erheblichen Limitation, da eine Volumenvergrößerung auch die Grenzen des Skale up's schnell erreichen läßt, da kontinuierlich neue Bedingungen erreicht werden. Diese sind auch dadurch bedingt, daß der durch die Rotation erreichte Umwurf dann zu höheren Druck- und Scherbelastungen der Zellen führt. Falls diese Rotationen nicht gemacht werden, ist andererseits eine gleichmäßige Versorgung der Zellen mit Sauerstoff nicht mehr gewährleistet. Dies ist durch die laterale Anordnung der gasdurchlässigen Folien/Membranen relativ zum Zellkultivierungsraum bedingt.

Die Einbringung von definierten Volumina für die Kammern des Kultursystems wird in US-PS 4,748,124 beschrieben. Hierfür wird die Kulturkammer in einem komprimierten Zustand gehalten. Dies hat den Nachteil, daß diese Kammern vor Beginn der Kultivierung in bestimmten Dimensionen festgelegt (definiert) werden müssen. Diese Definition des Kulturraums wird als ein Vorteil angesehen. Tatsächlich handelt es sich um einen entscheidenden Nachteil, da dadurch eine Anpassung an unterschiedliche Kulturphasen und ein Mitwachsen des Kultursystems bei 3-D Wachstum nicht möglich ist. Nur eine derartige Flexibilität ermöglicht jedoch den Einsatz des Systems für die Entwicklung künstlicher Organe aus einer kleinen Anzahl von Starterkulturen.

Zusätzlich wird in US-PS 4,748,124 die Einschränkung auf die Verwendung von Dialysemembranen vorgenommen. Dies ist von wesentlichen Nachteil für primäre Zellen wie z.B. Hepatozyten die Produkte und Katabolite, oder proteingebundene Toxine mit wesentlichem höheren Molekulargewicht transportieren (aufnehmen und abgeben müssen). Nur mikroporöse Membranen mit Proteinpermeabilität können dies gewährleisten.

Der vorliegenden Erfindung liegt die Aufgabe zugrunde eine Vorrichtung zu schaffen, mit der sehr variabel auf die sich ändernden Zustände, insbesondere Volumenänderungen (Erhöhung und Verkleinerung des Volumens) bei der Behandlung und/oder Züchtung von Zellen reagiert werden kann, wobei gleichzeitig auch eine Beobachtung möglich und darüber hinaus auch die Möglichkeit für eine Massenkultur gegeben sein soll.

Erfindungsgemäß wird diese Aufgabe durch die in Anspruch 1 genannten Merkmale gelöst.

Die erfindungsgemäße Vorrichtung ist durch ihren einfachen Aufbau für ein Massenkultursystem geeignet. Darüber hinaus läßt sie sich den jeweiligen Anforderungen anpassen. So kann z.B. beginnend mit einem sehr kleinen Volumen und einer geringen Zellenzahl, die in der Zellenkammer bzw. dem Zellkompartment behandelt oder kultiviert werden, diese auf ein Vielfaches des Anfangsvolumens vergrößert werden. Dies bedeutet man kann z.B. mit einer geringen Menge von zu untersuchendem Material beginnen und dieses dann in der Zellenkammer wachsen lassen. Auf diese Weise wird insbesondere bei teuren Substanzen auch eine Kosteneinsparung erreicht.

Durch die Möglichkeit über den stabilen Träger Luft- bzw. Sauerstoff zuzuführen, der über die gaspermeablen Folien in die Zellenkammer diffundieren kann, können ein oder mehrere derartige Einheiten sandwichartig übereinander in einen konventionellen Wärmeschrank gestellt werden. Aufgrund der Kombination der Trägerstruktur mit der Oxygenation über die gaspermeablen Folien sind keinerlei Pumpvorrichtungen für die Sauerstoff- und Kohlendioxyd-Versorgung erforderlich, womit die Handhabung erleichtert wird.

Von Vorteil ist weiterhin auch, daß bei der erfindungsgemäßen Vorrichtung die Zellenkammer auf einfache Weise stets beobachtet werden kann.

Eine sehr vorteilhafte und nicht naheliegende Weiterbildung der Erfindung ist in Anspruch 2 aufgezeigt.

Während man in der unteren Zellenkammer Zellen behandelt und/oder kultiviert kann die obere Kammer zur Zuführung von Nährmedium vorgesehen sein, das durch die mikroporöse Folie bzw. Membran in die Zellenkammer diffundiert. Hierzu muß die zweite Folie entsprechend mikroporös und flüssigkeitsdurchlässig sein. Ein weiterer Vorteil einer Nährmediumzuführung auf diese Art besteht darin, daß für die Zellen in der Zellenkammer ein deutlich geringerer Scherstreß gegeben ist. Die Zellen können unterhalb und oberhalb in Kollagen eingelagert werden und von oben her wird dann Nährmedium zugeführt.

Eine weitere Möglichkeit zur Nutzung der zweiten Kammer besteht darin, daß man diese auch zu Produktion bzw. "Ernte" von Substanzen verwenden kann, die in der Zellenkammer gebildet werden. So kann man z.B. weiße Blutzellen in der Zellenkammer kultivieren, setzt diese einem Antigen aus, wodurch die Zellen beginnen Antikörper zu produzieren. Diese Antikörper diffundieren dann durch die zweite Folie in die zweite Kammer und können von dort aus abgezogen werden. Auf diese Weise bleiben die Zellen in der Zellenkammer erhalten. Dabei ist lediglich dafür zu sorgen, daß die zweite Folie zwischen der Zellenkammer und der zweiten Kammer eine entsprechende Mikroporosität besitzt, so daß nur die gewünschten Substanzen durch die Folie perfundieren können.

Von Vorteil ist weiterhin auch, daß das in der zweiten Kammer sich befindende Medium ohne Störung der in der Zeilenkammer sich befindenden Zellen ausgetauscht werden kann. Gleiches gilt selbstverständlich auch umgekehrt. Auf diese Weise erhält man ein längerfristiges regenerationsfähiges System.

Vorteilhafte Weiterbildungen und Ausgestaltungen ergeben sich aus den übrigen Unteransprüchen und aus dem nachfolgend anhand der Zeichnung prinzipmäßig beschriebenen Ausführungsbeispiel.

Es zeigt:

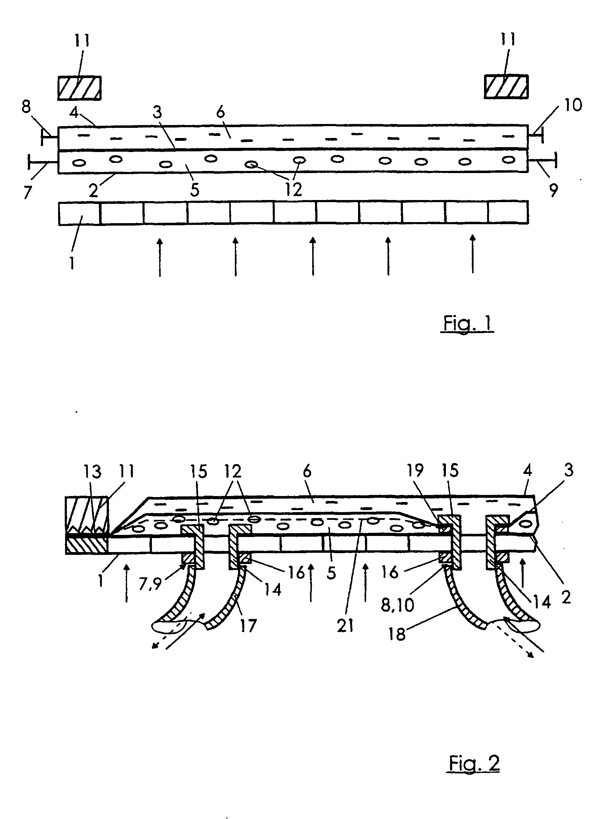

- Fig. 1: eine schematische Explosionsdarstellung der erfindungsgemäßen Vorrichtung in Seitenansicht,
- Fig. 2: ausschnittsweise eine vergrößerte Darstellung in einem Vertikalschnitt,
- Fig. 3: eine schematische Darstellung der erfindungsgemäßen Vorrichtung in einer Abwandlung, und
- Fig. 4: ein Kopfglied eines Rohrteiles einer Zulauf- oder einer Ablaufleitung in vergrößerter Darstellung in der Draufsicht.

Auf einen Träger 1 in Form einer Trägerplatte in Gitter-, Loch-, Profil- oder Wabenstruktur, wird eine elastische gasdurchlässige Teflonfolie 2 aufgelegt. Über die Teflonfolie 2 wird eine zweite Folie in Form einer porösen flüssigkeitsdurchlässigen Folie 3 aufgelegt. Über die Folie 3 wird eine gaspermeable Teflonfolie 4 gelegt. Die drei Folien sind an ihren Rändern abgedichtet. Auf diese Weise bildet sich zwischen der auf der unteren Fläche durchgehend unterstützten Teflonfolie 2 und der zweiten Folie 3, die mikroporös ist, eine variable und nach oben frei expansionsfähiger Zellenkammer 5 und zwischen der zweiten Folie 3 und der dritten Folie 4 eine variable, und nach oben frei expansionsfähige zweite Kammer 6. Die zweite Kammer 6 besitzt jeweils mindestens eine Zulaufleitung 7 bzw. 8 und eine Ablaufleitung 9 bzw. 10. Um eine gleichmäßige Verteilung von einzubringenden Substanzen und eine gleichmäßige Durchströmung zu erreichen, wird man die Zu- und Abläufe einer Kammer gegenüberliegend, am besten diagonal gegenüberliegend, anordnen. Für die Zellenkammer 5 reicht grundsätzlich ein Anschluß, mehrere sind jedoch möglich.

Als Abdeckung und zur Lagedefinierung können auf die dritte Folie 4 ein Rahmen oder mehrere Abstandshalter 11 aufgelegt werden. Der stabile Träger 1, die Zellenkammer 5, die zweite Kammer 6, die als Nährmediumkammer oder Sammelkammer dienen kann, und Rahmen bzw. Abstandshalter 11 bilden eine Einheit, wobei beliebige derartige Einheiten übereinander stapelbar sind. Sauerstoff kann von unten her durch den Träger 1 in Pfeilrichtung und von der Seite her zwischen die Abstandshalter 11 zur Versorgung von in die Zellenkammer 5 eingebrachten Zellen 12 dienen. In gleicher Weise kann Nährmedium aus der zweiten Kammer 6 durch die dialyseartige bzw. mikroporöse Folie 3 in die Zellenkammer 5 perfundieren.

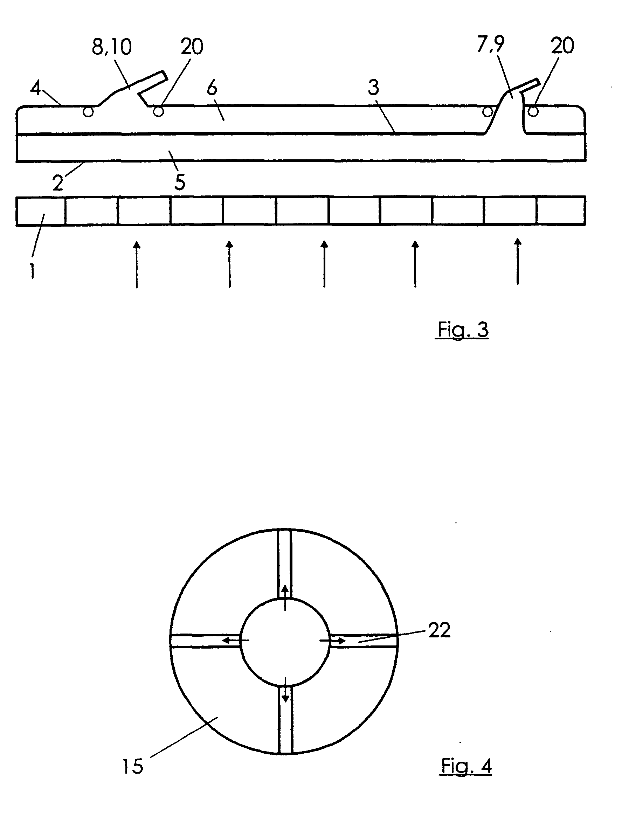

Aus der Fig. 2 ist ersichtlich, wie die Zellenkammer 5 und die zweite Kammer 6 gebildet werden und wie die Folien 2, 3 und 4 an ihren Rändern abgedichtet werden, um die beiden Kammern zu bilden. Hierzu kann der Rahmen bzw. können leistenartige Abstandshalter 11 auf ihrer Unterseite mit einer sägezahnartigen Struktur 13 als Klemm- bzw. Dichtglied versehen sein. Die drei Folien 2, 3 und 4 werden auf den Trägerrahmen 1 gelegt und nach Aufsetzen der Abstandshalter 11 durch die sägezahnartige Struktur 13 festgeklemmt, wenn die Abstandshalter 11 in nicht näher dargestellter Weise mit dem Träger 1 verbunden werden. Dies kann z.B. mit Schraubverbindungen oder durch Verkleben erfolgen. Auf diese Weise erfolgt umlaufend eine Abdichtung.

Die Folien 2,3,4 können auch verklebt bzw. verschweißt sein. Vor dem Aufsetzen des Rahmens bzw. der Abstandshalter 11 sind jedoch die Zuleitungen 7 und 8 und die Ablaufleitungen 9 und 10 einzubringen. Die Zu- und Ablaufleitungen können als vorgefertigte Einheiten ausgebildet sein. Um eine Verbindung nach außen herzustellen, können sie jeweils ein Rohrteil 14 mit einem ringförmigen Kopfglied 15. Das Rohrteil 14 besitzt ein Außengewinde, auf das eine Gewindemutter 16 aufschraubbar ist. Sobald die Teflonfolie 2 auf den Träger 1 aufgesetzt ist, wird jeweils mindestens eine Zulaufleitung 7 mit dem Rohrteil 14 und eine Ablaufleitung 9, ebenfalls mit dem Rohrteil 14 gleicher Bauart, durch die Membrane 2 durchgestoßen und jeweils in eine Bohrung des Trägers 1 eingesetzt. Dabei liegt das Kopfglied 15 auf der Oberseite des Trägers 1 an und die Folie 2 wird darunter geklemmt, wobei gegebenenfalls noch ein Dichtring (O-Ring) vorgesehen sein kann. Schraubt man nun von unten her die Gewindemutter 16 auf, so wird eine Abdichtung erreicht. Abschließend lassen sich Schläuche 17 bzw. 18 auf die Rohrteile 14 aufschieben. Auf diese Weise kann die Zellenkammer 5 befüllt oder entleert werden.

Nach dem Aufsetzen der mikroporösen Folie 3 wird diese ebenfalls durch das Rohrteil 14 durchstochen, welches dann ebenfalls durch eine Bohrung in dem Träger 1 gesteckt wird. Das Rohrteil 14 stellt für die zweite Kammer 6 entweder die Zulaufleitung oder die Ablaufleitung dar, sofern man nicht nur eine gemeinsame Leitung für Zu- und Ablauf vorsieht.

In vorteilhafter Weise wird man zwischen dem Kopfglied 15 und dem Träger 1 noch einen Dichtring 19 vorsehen. Mit dieser Ausgestaltung ist ebenfalls eine Zulauf- und Ablaufmöglichkeit für in die zweite Kammer einzubringendes Medium vorhanden.

Zu Beginn der Behandlung liegen die drei Folien 2, 3 und 4 direkt aufeinander. Wird nun Medium in die Zellenkammer 5 eingegeben, so kann sich aufgrund der Elastizität der Folie 3 und gegebenenfalls auch der Folie 2 ein entsprechender variabler Abstand einstellen, womit eine entsprechende Behandlungskammer bzw. Zellenkammer 5 geschaffen wird. Gleiches gilt für die zweite Kammer 6, die ebenfalls dadurch gebildet wird, daß über die Zulaufleitung 8 ein Medium, z.B. Nährmedium, eingebracht wird. In diesem Falle dehnt sich neben der Folie 3 auch die Folie 4 und gibt genügend Raum für die damit zu bildende zweite Kammer 6. Da nach oben keine besonderen Begrenzungen gegeben sind, können sich die Volumina in der Zellenkammer 5 und der zweiten Kammer 6 beliebig nach oben ausdehnen. Ausgehend von einem "Nullabstand" kann man Höhen von 1 bis 2 cm erreichen.

Kultiviert man z.B. in der Zellenkammer 5 Knochenmark, so ist eine Volumenvermehrung um den Faktor 100-1000 möglich. Man beginnt z.B. mit 100 Mikroliter bis 5 ml, wenn man nur einen dünnen Film aus Zellen 12 besitzt. Die Zellen wachsen dann in der Zellenkammer 5,durch Vermehrung der hämatopoetischen Stammzellen. Da Wachstumsfaktoren sehr teuer sind, bedeutet dies bei der erfindungsgemäßen Vorrichtung, daß man mit kleinen Mengen anfangen kann und auf diese Weise eine entsprechende Kosteneinsparung erreicht. Trotzdem ist es nicht erforderlich Unterbrechungen in der Behandlung oder einen Wechsel der Vorrichtung vorzunehmen, da das System ja mit der Behandlung mitwachsen kann. Zusätzlich ermöglicht das Bauprinzip, daß Knochenmarksstromazellen bei der Vermehrung der Stammzellen im Bioreaktor verbleiben können

Die erfindungsgemäße Vorrichtung kann z.B. auch zur Herstellung von Antikörper bzw. Impfstoffen verwendet werden. In diesem Falle gibt man in die Zellenkammer 5 immunaktivierende Virus- oder Bakterienbestandteile ein, die sich bildenden Klone produzieren Antikörper, die durch die Folie 3 diffundieren und können dann ohne Störung in der Zellenkammer 5 aus der zweiten Kammer 6 "geerntet" werden. Die Zellenkammer 5 kann im Bedarfsfalle auch noch unterteilt sein.

Eine Beobachtung der Vorgänge in der zweiten Kammer 6 und/oder der Zellenkammer 5 ist auf einfache Weise mit der erfindungsgemäßen Vorrichtung möglich. Man kann diese nämlich problemlos unter ein Mikroskop schieben und zwar ohne daß die Kultivierung in den Kammern davon beeinflußt wird. Praktisch läßt sich auf diese Weise jede einzelne Einheit, bzw. jedes Modul beobachten.

Statt einer Klemmung der Folien 2, 3 und 4 wie in der Fig. 2 dargestellt, können diese auch seitlich bzw. umlaufend auf den Träger 1 aufgeklebt werden.

Je nach Zellenart und/oder Kultivierungsform kommt man gegebenenfalls auch mit nur einer einzigen Kammer, in diesem Falle der Zellenkammer 5 aus, und mit nur zwei Folien, nämlich der Folie 2 und 3. So kann man z.B. in der Zellenkammer 5 Pilze züchten, die aufgrund der Elastizität der Folie 3 und gegebenenfalls auch der Folie 2 mit entsprechender Volumenvergrößerung der Zellenkammer 5 wachsen können.

Es kann auch eine Zwischenfolie 21 verwendet werden (in der Fig. 2 gestrichelt in der Zellenkammer 5 dargestellt), wodurch zwei Zellenkammerabschnitte entstehen.Die zweite Folie 11 kann eventuell ebenfalls mikroporös sein.

Einer der wesentlichen Vorteile der erfindungsgemäßen Vorrichtung besteht darin, daß aufgrund des Mitwachsens der Zellenkammer 5 und der zweiten Kammer 6 bei einem Ernten von Substanzen aus der zweiten Kammer 6 die Zellen in der Zellenkammer 5 nicht wie beim Stand der Technik darunter leiden bzw. mit entfernt werden müssen und damit verloren sind.

Dies bedeutet z.B., daß bei der Expansion von Knochenmark die hämatopoetischen Stammzellen expandieren können und zugleich die Stromazellen über lange Zeiträume im Bioreaktor verbleiben, d.h. bis die genügende Volumenvermehrung der Stammzellen (z.B. 500 ml von 1 bis 5 ml) erreicht ist.

Von Vorteil ist weiterhin auch, daß nach Beendigung einer Versuchsreihe von der Einheit zumindest der Träger 1 und der Rahmen bzw. die Abstandshalter 11 wieder verwendbar sind. Es ist lediglich erforderlich die beiden Kammern, nämlich die Zellenkammer 5 und die zweite Kammer 6, die aus den Folien 2, 3 und 4 gebildet sind und praktisch Beutel bilden, auszutauschen. Ebenso sind auch die Zulauf- und Ablaufleitungen 7 bis 10 wiederverwendbar.

Auf Grund der geringen Verfügbarkeit bestimmter Starterzellen (z.B. humanes Knochenmark) und/oder der enormen Kosten an Wachstumsfaktoren und Interleukinen soll das Volumen so gering als möglich sein (ca. 1 bis 5 ml), andererseits soll es aber im Kulturverlauf bis zu 100 bis 1000fach zunehmen zu können. So soll z.B. Knochenmark eines Patienten extrakorporal derart vermehrt werden, daß ca. 500 ml wieder zurücktransplantiert werden können.

Zusätzlich können spezifische Kultivierungsphasen bei z.B. Leberzellen erfordern, daß während der Anwachsphasen hohe Volumina (200 bis 300 ml, bei 1000 cm² Fläche) an Nährmedium in den durch die erfindungsgemäße Vorrichtung geschaffenen Bioreaktor eingebracht werden, um ein optimales Anwachsen der Zellen und eine Vermehrungsphase für die nachfolgenden 24 bzw. 48 Std. wartungsfrei unter nicht perfundierten Bedingungen zu ermöglichen. Gerade in der Anwachsphase der Zellen ist es notwendig störende Strömungen zu vermeiden. Wäre das Volumen eines Bioreaktors bereits initial auf sehr niedrige Volumina eingestellt, würde dies jedoch eine sehr verkürzte Toleranzzeit bei Stop der Perfusion bedeuten, da das Nährmedium innerhalb von Minuten verbraucht wäre. Zusätzlich würden toxische Produkte der Zellen, Katabolite und auch bei der Zellisolation primärer Zellen (z.B. Leber) durch die Zellisolation mittels Kollagenase anfallende Zellyse Produkte in hohen lokalen Konzentrationen das Anwachsen und damit den Start der Kultur behindern und bei sensiblen Zellen zum Absterben dieser Zellen führen.

Bei der Vorbereitung der Kultur primärer Leberzellen wird wie folgt vorgegangen:
Zuerst wird Kollagen in flüssiger Lösung z.B. mit in lmmol HCl und 1,5 mg/ml Kollagen Typ I mit 3 µg/ml Fibronektin in die Zellenkammer 5 bzw. das Zellkompartment injiziert und überschüssiges Kollagen sofort wieder entfernt. Dadurch kollabiert der Reaktor ohne zusätzliches Öffnen von Ventilen blasenfrei, da ein Vakuum gezogen wird. Diese Flexibilität ist somit auch steriltechnisch und kulturtechnisch von Vorteil, da Luftblasen für Zellen toxisch sind. Die Entfernung des überschüssigen Kollagens stellt auch eine Kostenersparnis dar, da dieses Kollagen somit auch für andere Module verwendet werden kann.

Der Reaktor bzw. die Vorrichtung kann so bei 37°C belassen werden, oder auch mit UV-Licht bestrahlt werden um eine Verfestigung des Kollagens (Crosslinking) zu erreichen. Das Kollagen kann mit oder ohne 10fach Konzentrat eines Nährmediums in die Zellenkammer 5 hinzugegeben werden, wodurch eine sofortige pH Neutralisierung ermöglicht wird. Wird dies einfachheitshalber nicht getan, so kann durch Zugabe eines hohen Nährmediumanteils auf Grund der Flexibilität der Vorrichtung auch dann noch eine pH Neutralisierung erfolgen, da der Verdünnungseffekt durch das Kulturmedium die hierzu notwendige Pufferung ermöglicht.

Üblicherweise wird bei der Ansaat der Hepatozyten eine Konzentration von 1 x 10⁶ Zellen pro ml eingestellt. Bei einer Kulturfläche von 1000 cm² würden deshalb bereits 200 ml erforderlich sein. Dieses Volumen wird nach 2 bis 4 h ausgetauscht um Toxine und Zelldetritus zu entfernen und frisches Nährmedium für weitere 24 bis 48 Std. auf den Hepatozyten belassen zu können. Danach wird das Medium vollständig entfernt, so daß nur eine einzelne, noch nicht konfluente Zellage, verbleibt. Diese wird dann mit ca. 20 bis 50 ml Kollagen im Mix in der Zellenkammer 5 mit einem 10fach Konzentrat eines Nährmediums (z.B. Williams E) überschichtet. Danach wird aus der Zellenkammer 5 überschüssiges Kollagen wieder abgesaugt. Dadurch kann die Zellenkammer 5 kollabieren bzw. zusammenfallen und sich die untere gaspermeable Folie bzw. Membran 2 mit den darauf angewachsenen Zellen mit der mittleren Folie 3 bzw. Membran annähern. Dies unterstützt die Festigkeit der Kollagenschicht und schützt vor Scherkrafteinfiüssen.

Nach Einbringung der zweiten Kollagenschicht (48 h nach der Zellaussaat) in die Zellenkammer 5 und Abwarten für ca. 1h, wird in die obere zweite Kammer Nährmedium eingefüllt.

Je höher der Volumengehalt an Nährmedium ist, desto problemloser läßt sich der Bioreaktor in einem stand by Betrieb halten.

Bei der Verwendung von primären Hepatozyten ist es entgegen der etablierten Meinung von Vorteil einen Anteil nicht parenchymaler Zellen von zumindest mehr als 1% (20 bis 30%) mit in die Kultur beizumischen, da diese Wachstumsfaktoren lokal produzieren und somit erst die Expansion der ansonsten nicht proliferierenden Hepatozyten spontan ermöglichen. Ansonsten müßten die Hepatozyten mit hohen Mengen an exogen zugeführten und sehr teuren Wachstumsfaktoren (z.B. EGF = epidermal growth factor , TGF = transforming growth factor oder HGF = hepatocyte growth factor) stimuliert werden. Durch GH = growth Hormon (Wachstumshormon) kann die Ausschüttung der endogenen (durch die NPC = Nicht-parenchymale Zellen) produzierten Zellen noch verstärkt werden und damit kostengünstig eine zelluläre Regeneration des Bioreaktors im längerfristigen Betrieb erreicht werden.

Soll das Reaktorsystem als bioartifizielles Organ (z.B. Leber) eingesetzt werden, so ist ein minimales Volumen notwendig, da bei reduzierten Schichtdicken des verwendeten Plasmas der Stoffaustausch durch Diffusion wesentlich effizienter ist und weil so Produkte des Reaktors nicht auf unphysiologische Konzentrationen verdünnt werden. Die vertikale Höhe der Perfusionskammer bzw. zweiten Kammer 6 soll hierbei nun wieder nur 10 bis 50 µm betragen.

Um den in vivo Einsatz bei Patienten im Leberversagen durchführen zu können, werden die Bioreaktorenmodule unter Verwendung von Sterilkonnektoren parallel geschaltet, das Kulturmedium über Mehrkanalpumpensysteme durch Absaugen vollständig entfernt und Plasma des Patienten (in Kombination mit einem Plasmaseparator und ein Reservoir) in möglichst dünnen Schichten durch die obere zweite Kammer 6 hindurchgeführt. Hierzu ist es vorteilhaft das Plasma wiederum mittels Saugen durch den Reaktor bzw. die Vorrichtung hindurchzuführen, da dieser dann auf das minimale Volumen kollabieren kann. Zur Unterstützung und zur Sicherheit im Perfusionbetrieb kann dann eine gleichgeschaltete Pumpe vor dem Reaktor die Aufrechterhaltung konstanter Perfusionsbedingungen im Reaktorbetrieb bei minimalen Schichtdicken ermöglichen.

Eine weitere Anwendung stellt die Kultur von Keratinozyten auf den gaspermeablen Filmen bzw. Folien wie z.B. Teflon dar. Bei geringen Schichtdicken dieser Filme (z.B. 25 µm) ergibt sich eine hohe Sauerstoffpermeabilität. Wenn, wie üblich, Keratinozyten auf Folien expandiert werden, ergibt sich bisher das Problem, daß bei Verwendung dieser Folien als Wundabdeckung (z.B. nach Verbrennungen und Entfernung der verbrannten Hautreste durch Escharektomie) die auf den Folien angewachsenen Zellen nach dem Umklappen der Folie auf die Wunde falsch orientiert sind, da die Verhornung immer auf der Luft exponierten Seite erfolgt.

Bei der Verwendung der gaspermeablen Folie 2 als Adhäsionssubstrat kommt es initial zu einer Adhäsion und Proliferation der Keratinozyten, zusätzlich kann ein mehrschichtiges Wachsen wie üblich erfolgen. Wird im Kulturverlauf die die Zellen überlagernde Mediumschichtdicke in der zweiten Kammer 6 dahingehend erhöht, daß Limitationen entsprechend der O₂-Versorgung entstehen, so kann eine Umorientierung der Zellen erfolgen, mit einer Verhornung an der unter Seite. Die O₂-Versorgung ist entsprechend dem Fickschen Diffusionsgesetz von der Schichtdicke des Mediums abhängig und O₂ tendiert bereits bei Schichtdicken von 1 mm perizellulär gegen 0.

Wird dann der Bioreaktor an dieser Seite bzw. die Folie 2 für diese Anwendung so konzipiert, daß wie bei einem Becher die Folie 2 an Sollbruchstellen bzw. vorgesehen Ablösungsflächen (nicht permanente Befestigung) abgezogen werden kann, so läßt sich hier sehr einfach eine sterile Wundabdeckung mit korrekt orienterten mehrschichtigen Keratinozytenkulturen erreichen.

Hierzu kann aus steriltechnischen Gründen der Bioreaktor eine zusätzliche und entfernbare sterile Ummantelung besitzen (Behältnis oder Extrafolie, gasdurchlässige Membrane oder Struktur, Papierverpackung oder Kunststoff mit Sterilfiltern, die Luft in die Vorrichtung hineinlassen).

Diese Variabilität des Bioreaktors, zeitlich versetzt hohe und niedrigste Volumina zu ermöglichen, ist ein wesentlicher Vorteil der Erfindung. Diese Flexibilität ermöglicht es auch derart unterschiedlliche Organ- und Zellsysteme zu kultivieren und diese in multizelluläre und mehrschichtige Kulturen zu expandieren.

Es handelt sich hierbei somit um ein Volumen-sensitives Bauprinizip das ein Verfahren ermöglicht, bei dem zeitlich fraktioniert unterschiedliche Volumina eingestellt werden können. Dies bedeutet auch, daß der Reaktor "mitwächst" und im Gegensatz zum Stand der Technik keinerlei ungewolltes "Totvolumen" besitzt.

Bei Kultivierung der Zellen in der unteren Zellenkammer 5 bzw. dem Kompartment und der dadurch unmittelbaren Nähe zur Sauerstoffversorgung ist es nicht unbedingt erforderlich, daß die obere Folie 4 gaspermeabel ist. Im allgemeinen wird man die Zellenkammer 5 auf dem Träger 1 anordnen. Es ist jedoch gegebenenfalls auch die umgekehrte Anordnung möglich, d.h. daß sich die Zellenkammer 5 oben befindet und die zweite Kammer unten, wobei in diesem Falle die zweite Kammer 6 auf dem Träger 1 zur Unterstützung aufliegt.

Die Anschlüsse bzw. Zu- und Ablaufleitungen 7 bis 10 können einfachheitshalber entweder alle nach oben oder nach unten ausgeleitet (oder gemischt) werden. Der Vorteil bei der Anbringung nach oben ist, daß bei der Herstellung (z.B. Tiefziehverfahren) diese gleich in die obere Folie 4 bzw. 3 integriert werden können (siehe Prinzipdarstellung in Fig. 3). Aus der Fig. 3 ist weiterhin auch ersichtlich, daß zur Vermeidung eines Ansaugens bzw. Aufeinanderklebens der Folien 2, 3 und 4 im Bereich der Zu- und Ablaufleitungen 7 bis 10 Abstandshalter 20 oder Abstandsnoppen vorgesehen sind. Ein gegenseitiges Verkleben bzw. Ansaugen kann auch dadurch verhindert werden, daß z.B. in den Kopfgliedern 15 der Rohrteile 14 von der Zulauf- bzw. Rücklauföffnung aus radial nach außen führende Kanäle eingeformt sind, wodurch auch bei einem direkten Aufeinanderliegen der Folien bzw. einer Ansaugung sichergestellt ist, daß Medium in die jeweilige Kammer eingebracht werden kann.

Der Bioreaktor bzw. die Vorrichtung kann auch so gebaut werden, daß die Kultureinheit als Beutel mit den Folien 2,3,4 und mit den entsprechenden Anschlüssen, nebst daraus gebildeten Kammern 5 und 6, erstellt wird. Der Bioreaktor bzw. die Kultureinheit kann dann auf dem unterstützenden Träger 1 aufgebracht und fixiert werden. Dies kann durch Einspannen mittels seitlicher Löcher im Beutel geschehen.

Die Verwendung allein des Beutels würde die Funktionalität des Bioreaktors nicht ermöglichen, da nur in der Kombination mit einer Unterstützungsstruktur bzw. dem Träger 1 die geforderte Flexibilität und das einfache Handling erreicht wird. Die Befüllung des Beutels ohne die durchgehende Unterstützungsstruktur bzw. den Träger 1 würde sofort zu einem Versakken der Medien bzw. Zellen am tiefsten Punkt führen und damit zum Absterben der Zellen.

Idealerweise liegt die Porengröße der Folie 3 bei 0,2 µm (Sterilfiltrationsgrenze), wodurch zusätzlich ein Sterilschutz der Zellenkammer 5 erreichbar ist. Die Verwendung höherer oder niedriger Porengrößen ist jedoch möglich. Diese können so klein gewählt werden, daß z.B. Viren, die für Transfektionsexperimente an z.B. Hepatozyten oder Knochenmarkszellen oder Keratinozyten verwendet werden, in der entsprechenden Zellenkammer 5 zurückgehalten werden.

Als Folienmaterial kann z.B. Teflon, Silikon, Polycarbonat oder Polyester verwendet werden. Für die mikroporöse Folie 3 ist insbesondere Polycarbonat oder Polyester wegen der Transparenz besonders geeignet.
Die Oberflächen der Folien und Schläuche können z.B. mit Heparin vorbeschichtet sein,damit eine Complement-Aktivierung reduziert wird.

Im Unterschied zu Hohlfasersystemen ist eine Entfernung der als Schichten wachsenden Zellen nach z.B. auch Abtrypsinierung oder durch einfaches Absaugen sehr einfach möglich.

Mit der erfindungsgemäßen Vorrichtung lassen sich auch Pflanzenzellen oder Algen behandeln, um damit z.B. Sauerstoff zu produzieren, die in den transparenten Bioreaktoren Photosynthese betreiben können. Dies könnte z.B. in der Raumfahrt benützt werden, um CO₂ zu absorbieren und O₂ bereitzustellen.

Weiterhin könnten derartige Systeme bei der Verwendung von Bakterien zur Entgiftung mit Gasen verunreinigter Atmosphären eingesetzt werden.

## Patentansprüche

1. Vorrichtung zum Züchten und/oder Behandeln von Zellen mit folgenden Merkmalen:
1.1 auf einem gasdurchlässigen Träger (1) ist eine erste Kammer (Zellenkammer 5) angeordnet,
1.2 die erste Kammer (Zellenkammer 5) ist durch eine erste gaspermeable, nicht flüssigkeitsdurchlässige Folie (2) gebildet, die auf den Träger (1) aufgelegt ist, und eine zweite mikroporöse Folie (3), die über der ersten Folie (2) angeordnet oder mit dieser einstückig ist,
1.3 wenigstens eine der beiden Folien (2,3) ist derart elastisch, daß das Endvolumen der ersten Kammer (5) ein Vielfaches seines Ausgangsvolumens einnehmen kann und
1.4 die erste Kammer (5) ist mit wenigstens einer Zuund/oder Ablaufleitung (7,9) versehen.

2. Vorrichtung nach Anspruch 1,
**dadurch gekennzeichnet, daß**
über der zweiten Folie (3) eine dritte Folie (4) angeordnet und mit der zweiten Folie (3) verbunden oder einstückig mit dieser ist, wobei die zweite Folie (3) und/oder die dritte Folie (4) derart elastisch ist bzw. sind, daß zwischen der zweiten Folie (3) und der dritten Folie (4) eine zweite Kammer (6) herstellbar ist, deren Endvolumen ein Vielfaches seines Ausgangsvolumens einnehmen kann, wobei die zweite Folie (3) als mikroporöse Folie ausgebildet ist, die den Durchtritt von ausgewählten Substanzen aus der ersten Kammer (Zellenkammer 5) in die zweite Kammer (6) zuläßt, wobei die zweite Kammer (6) mit wenigstens einer Zulauf- und/oder Ablaufleitung (9,10) versehen ist.

3. Vorrichtung nach Anspruch 1 oder 2,
**dadurch gekennzeichnet, daß**
eine Vielzahl von aus ersten und zweiten Folien (2,3) gebildeten Zellenkammern (5) und aus zweiten und dritten Folien (3,4) gebildeten zweiten Kammern (6) übereinander legbar sind.

4. Vorrichtung nach einem der Ansprüche 1 bis 3,
**dadurch gekennzeichnet, daß**
über der dritten Folie (3,4) ein Rahmen (11) gesetzt ist.

5. Vorrichtung nach Anspruch 4,
**dadurch gekennzeichnet, daß**
der Rahmen (11) auf seiner Unterseite mit Klemmund/oder Dichtgliedern (13) für die Folien (2,3,4) versehen ist.

6. Vorrichtung nach einem der Ansprüche 1 bis 4,
**dadurch gekennzeichnet, daß**
die Folien an ihren Rändern flüssigkeitsdicht mit dem Träger (1 und/oder untereinander verbunden sind.

7. Vorrichtung nach Anspruch 6,
**dadurch gekennzeichnet, daß**
die Folien (2,3,4) auf den Träger (1) aufgeklebt sind.

8. Vorrichtung nach einem der Ansprüche 1 bis 7
**dadurch gekennzeichnet, daß**
die Zu- und Ablaufleitungen (7,8,9,10) als vorgefertigte Einheiten ausgebildet sind, die durch die jeweilige dazugehörige Folie (2,3,4) gesteckt, in dieser befestigt und an dem Träger (1) befestigbar sind.

9. Vorrichtung nach Anspruch 8
**dadurch gekennzeichnet, daß**
die vorgefertigten Einheiten durch Halteverbindungen mit dem Träger (1) verbindbar sind.

10. Vorrichtung nach Anspruch 8 oder 9
**dadurch gekennzeichnet, daß**
jede vorgefertigte Einheit ein Rohrteil (14) mit einem Gewindeabschnitt aufweist, auf den eine Gewindemutter (16) aufschraubbar ist, wobei der Träger (1) zwischen einem Kopfglied des Rohrteiles (14) und der Gewindemutter (16) eingeklemmt ist.

11. Vorrichtung nach Anspruch 10,
**dadurch gekennzeichnet, daß**
zwischen dem Kopfglied (15) und dem Träger (1) und/oder einer Folie (2 bzw. 3) Dichtringe oder Abstandshalter (19) angeordnet sind.

12. Vorrichtung nach einem oder mehreren der vorhergehenden Ansprüche,
**dadurch gekennzeichnet, daß**
die Folien (2,3,4) aus Teflon, Silikon, Polycarbonat oder Polyester bestehen.

13. Vorrichtung nach einem oder mehreren der vorhergehenden Ansprüche,
**dadurch gekennzeichnet, daß**
der Träger (1) als Gitter- oder Lochprofilplatte ausgebildet ist.

14. Vorrichtung nach einem oder mehreren der vorhergehenden Ansprüche,
**dadurch gekennzeichnet, daß**
die Zu- und Ablaufleitungen (7,8,9,10) in die dritte Folie und die zweite Folie als Einheit integriert sind.

15. Vorrichtung nach einem oder mehreren der vorhergehenden Ansprüche,
**dadurch gekennzeichnet, daß**
die Zellenkammer (5) durch eine Zwischenfolie (21) in zwei Abschnitte unterteilt ist.

16. Verfahren zum Züchten und/oder Behandeln von Zellen in folgenden Schritten:
16.1 auf einen gasdurchlässigen Träger (1) wird eine erste gaspermeable, nicht flüssigkeitsdurchlässige Folie (2) gelegt,
16.2 auf die erste Folie (2) wird eine zweite mikroporöse Folie (3) gelegt, wobei wenigstens eine der beiden Folien (2,3) elastisch ist,
16.3 auf die zweite Folie (3) wird eine dritte Folie (4) gelegt, wobei die zweite oder die dritte Folie (3 bzw. 4) elastisch ist,
16.4 die Folien (2,3 und 4) werden an den Rändern abgedichtet,
16.5 zwischen die erste Folie (2) und die zweite Folie (3) werden Zellen (12) zum Züchten und/oder Behandeln eingebracht,
16.6 zwischen die zweite und die dritte Folie (3,4) wird ein zweites Medium eingebracht, und
16.7 die Volumina in den beiden auf diese Weise gebildeten Kammern (5,6) werden entsprechend den gestellten Forderungen variiert.

## Claims

1. Device for culturing and/or treating cells having the following features:
1.1 a first chamber (cell chamber 5) is arranged on a gas-permeable carrier (1),
1.2 the first chamber (cell chamber 5) is formed by a first gas-permeable, non-liquid-permeable film (2) which is laid on the carrier (1), and a second microporous film (3), which is arranged over the first film (2) or is integral with this film,
1.3 at least one of the two films (2, 3) is elastic such that the final volume of the first chamber (5) can be many times its starting volume, and
1.4 the first chamber (5) is provided with at least one supply and/or discharge line (7, 9).

2. Device according to Claim 1,
**characterized in that** a third film (4) is arranged over the second film (3) and is connected to the second film (3) or is integral with this film, with the second film (3) and/or the third film (4) being elastic such that a second chamber (6) can be formed between the second film (3) and the third film (4), the final volume of which chamber can be many times its starting volume, with the second film (3) being a microporous film which permits the passage of selected substances from the first chamber (cell chamber 5) into the second chamber (6), with the second chamber (6) being provided with at least one supply and/or discharge line (9, 10).

3. Device according to Claim 1 or 2, **characterized in that** a multiplicity of cell chambers (5) which are formed from first and second films (2, 3) and second chambers (6) which are formed from second and third films (3, 4) can be laid one above the other.

4. Device according to one of Claims 1 to 3, **characterized in that** a frame (11) is placed over the third film (3, 4).

5. Device according to Claim 4, **characterized in that** the frame (11) is provided, on its underside, with clamping and/or sealing members (13) for the films (2, 3, 4).

6. Device according to one of Claims 1 to 4, **characterized in that** the films are connected, at their edges and in a liquid-tight manner, to the carrier (1) and/or to each other.

7. Device according to Claim 6, **characterized in that** the films (2, 3, 4) are glued to the carrier (1).

8. Device according to one of Claims 1 to 7, **characterized in that** the supply and discharge lines (7, 8, 9, 10) are prefabricated units which are inserted through the respective pertinent film (2, 3, 4) and affixed in this film, and can be affixed to the carrier (1).

9. Device according to Claim 8, **characterized in that** the prefabricated units can be connected to the carrier (1) by means of holding connections.

10. Device according to Claim 8 or 9, **characterized in that** each prefabricated unit possesses a pipe part (14) having a thread section onto which a thread nut (16) can be screwed, with the carrier (1) being clamped between a head member of the pipe part (14) and the thread nut (16).

11. Device according to Claim 10, **characterized in that** sealing rings or spacers (19) are arranged between the head member (15) and the carrier (1) and/or a film (2 or 3, respectively).

12. Device according to one or more of the preceding claims, **characterized in that** the films (2, 3, 4) consist of Teflon, silicone, polycarbonate or polyester.

13. Device according to one or more of the preceding claims, **characterized in that** the carrier (1) is in the form of a gridded or hole-profiled plate.

14. Device according to one or more of the preceding claims, **characterized in that** the supply and discharge lines (7, 8, 9, 10) are integrated as a unit into the third film and the second film.

15. Device according to one or more of the preceding claims, **characterized in that** the cell chamber (5) is subdivided into two sections by means of an intermediate film (21).

16. Process for culturing and/or treating cells in the following steps:
16.1 a first gas-permeable, nonliquid-permeable film (2) is laid on a gas-permeable carrier (1),
16.2 a second microporous film (3) is laid on the first film (2), with at least one of the two films (2, 3) being elastic,
16.3 a third film (4) is laid on the second film (3), with the second or the third film (3 and 4, respectively) being elastic,
16.4 the films (2, 3 and 4) are sealed at their edges,
16.5 cells (12) are introduced, for culturing and/or treatment, between the first film (2) and the second film (3),
16.6 a second medium is introduced between the second and the third film (3, 4), and
16.7 the volumes in the two chambers (5, 6) which are formed in this way are varied in accordance with the requirements which are placed on them.

## Revendications

1. Dispositif pour cultiver et/ou manipuler des cellules, comportant des caractéristiques suivantes :
1.1 une première chambre (chambre à cellules 5) est disposée sur un support perméable aux gaz (1),
1.2 la première chambre (chambre à cellules 5) est formée par une première pellicule (2) perméable aux gaz, imperméable aux liquides, qui est posée sur le support (1), et par une deuxième pellicule microporeuse (3) qui est disposée au-dessus de la première pellicule (2) ou qui est d'une seule pièce avec celle-ci,
1.3 au moins une des deux pellicules (2, 3) est élastique de telle manière que le volume final de la première chambre (5) puisse occuper un multiple de son volume de départ et
1.4 la première chambre (5) est pourvue d'au moins une conduite d'arrivée et/ou d'une conduite d'écoulement (7, 9).

2. Dispositif selon la revendication 1,
**caractérisé en ce que**
une troisième pellicule (4) est disposée au-dessus de la deuxième pellicule (3) et est reliée à la deuxième pellicule (3) ou est d'une seule pièce avec celle-ci, la deuxième pellicule (3) et/ou la troisième pellicule (4) étant élastique(s) de telle manière qu'il soit possible de former entre la deuxième pellicule (3) et la troisième pellicule (4) une deuxième chambre (6) dont le volume final peut occuper un multiple de son volume de départ, la deuxième pellicule (3) étant constituée par une pellicule microporeuse qui admet le passage de substances sélectionnées de la première chambre (chambre à cellules 5) à la deuxième chambre (6), la deuxième chambre (6) étant pourvue d'au moins une conduite d'arrivée et/ou d'une conduite d'écoulement (9, 10).

3. Dispositif selon la revendication 1 ou 2,
**caractérisé en ce que**
une pluralité de chambres à cellules (5) formées de premières et deuxièmes pellicules (2, 3) et de deuxièmes chambres (6) formées de deuxièmes et troisièmes pellicules (3, 4) peuvent être disposées l'une au-dessus de l'autre.

4. Dispositif selon l'une des revendications 1 à 3,
**caractérisé en ce que**
un cadre (11) est posé par-dessus la troisième pellicule (3, 4).

5. Dispositif selon la revendication 4,
**caractérisé en ce que**
le cadre (11) est pourvu, sur son côté inférieur, d'organes de serrage et/ou d'étanchéité (13) pour les pellicules (2, 3, 4).

6. Dispositif selon l'une des revendications 1 à 4,
**caractérisé en ce que**
les pellicules sont reliées au support (1) et/ou entres elles à joint étanche aux liquides le long de leurs bords.

7. Dispositif selon la revendication 6,
**caractérisé en ce que**
les pellicules (2, 3, 4) sont collées sur le support (1).

8. Dispositif selon l'une des revendications 1 à 7,
**caractérisé en ce que**
les conduites d'arrivée et d'écoulement (7, 8, 9, 10) sont constituées par des unités préfabriquées qui sont enfilées chacune à travers la pellicule respective (2, 3, 4), et fixées dans cette dernière, et qui peuvent être fixées au support (1).

9. Dispositif selon la revendication 8,
**caractérisé en ce que**
les unités préfabriquées peuvent être assemblées au support (1) par des assemblages de retenue.

10. Dispositif selon la revendication 8 ou 9,
**caractérisé en ce que**
chaque unité préfabriquée comporte une partie tubulaire (14) présentant un segment fileté sur lequel un écrou fileté (16) peut être vissé, le support (1) étant serré entre un élément de tête de la partie tubulaire (14) et l'écrou fileté (16).

11. Dispositif selon la revendication 10,
**caractérisé en ce que**
des bagues d'étanchéité ou des entretoises (19) sont disposées entre l'élément de tête (15) et le support (1) et/ou une pellicule (2, respectivement 3).

12. Dispositif selon l'une ou plusieurs des revendications précédentes,
**caractérisé en ce que**
les pellicules (2, 3, 4) sont faites de Téflon, de silicone, de polycarbonate ou de polyester.

13. Dispositif selon l'une ou plusieurs des revendications précédentes,
**caractérisé en ce que**
le support (1) est formé d'une plaque profilée en forme de grille ou perforée.

14. Dispositif selon l'une ou plusieurs des revendications précédentes,
**caractérisé en ce que**
les conduites d'arrivée et d'écoulement (7, 8, 9, 10) sont intégrées dans la troisième pellicule et dans la deuxième pellicule pour former une unité.

15. Dispositif selon l'une ou plusieurs des revendications précédentes,
**caractérisé en ce que**
la chambre à cellules (5) est divisée en deux compartiments par une pellicule intermédiaire (21).

16. Procédé pour cultiver et/ou manipuler des cellules dans les étapes suivantes :
16.1 on pose une première pellicule (2) perméable aux gaz, imperméable aux liquides sur un support (1) perméable aux gaz,
16.2 on pose une deuxième pellicule microporeuse (3) sur la première pellicule (2), au moins une des deux pellicules (2, 3) étant élastique.
16.3 on pose une troisième pellicule (4) sur la deuxième pellicule (3), la deuxième ou la troisième pellicule (3, respectivement 4) étant élastique,
16.4 on ferme les pellicules (2, 3 et 4) à joint étanche le long des bords,
16.5 on introduit des cellules (12) qu'il s'agit de cultiver et/ou de manipuler entre la première pellicule (2) et la deuxième pellicule (3),
16.6 on introduit un deuxième milieu entre les deuxième et troisième pellicules (3, 4), et
16.7 on fait varier les volumes intérieurs des deux chambres (5, 6) qui sont formées de cette façon en fonction des exigences qui se présentent.
